# EUROPEAN PATENT APPLICATION

(11) **EP 1 568 359 A1**
(43) Date of publication of application: **31.08.2005**
(21) Application number: 03777182.1
(22) Date of filing: 03.12.2003
(51) Int. Cl.: A61K 9/06, A61K 9/10, A61K 47/12, A61K 47/34, A61K 47/38, A61K 31/573, A61P 27/02, A61P 43/00

(54) **DRUG DELIVERY SYSTEM USING SUBCONJUNCTIVAL DEPOT**

(30) Priority: 04.12.2002 JP 2002352230
(71) Applicant: SANTEN PHARMACEUTICAL CO., LTD., Osaka 533-8651 (JP)
(72) Inventor: YAMADA, Kazuhito c/o SANTEN PHARMACEUTICAL CO. LTD, Ikoma-shi, Nara 630-0101 (JP); KUWANO, Mitsuaki c/o SANTEN PHARMACEUTICAL CO. LTD, Ikoma-shi, Nara 630-0101 (JP)
(74) Representative: Peaucelle, Chantal
(86) International application number: PCT/JP2003/015450
(87) International publication number: WO 2004/050060

(57) **Abstract**

The present invention provides a drug delivery system to a posterior segment wherein a pharmaceutical composition comprising a drug and a vehicle is administered subconjunctivally to form a depot out of the vehicle, and thereby the drug is gradually released from the depot to enable an effective concentration of the drug to be maintained, the pharmaceutical composition comprising the vehicle which is in the form of gel subconjunctivally and the drug suspended in the vehicle.

## Description

### Technical Field

The present invention relates to a drug delivery system (hereinafter abbreviated as "DDS") to posterior segments of the eye such as a retina, a choroid, an optic nerve, a vitreous body and a crystalline lens.

### Background Art

Diseases of posterior segments of the eye such as a retina, a choroid, an optic nerve, a vitreous body and a crystalline lens are often intractable, and a development of an effective pharmacotherapy is eagerly desired. Though ophthalmopathy is most generally treated by instillation of drugs, the drugs are hardly delivered to the posterior segments of the eye such as a retina, choroid, an optic nerve, a vitreous body and a crystalline lens. Even if the drugs are delivered to the posterior segments of the eye, it is very difficult to maintain a drug concentration in those tissues.

In view of this, an intravenous injection, oral administration and a intravitreous injection are attempted to administer the drugs for the diseases of the posterior segments of the eye. However, the intravenous injection and the oral administration can deliver only a very small amount of drugs to the posterior segments of the eye which are target sites, and sometimes causes unexpected strong systemic actions (side effects) of the drugs.

In the case of the intravitreous injection, since the drug is directly injected into the eye, the amount of the drug to be delivered to the posterior segments of the eye is larger than those of the intravenous injection and the oral administration. The drug delivery to the posterior segments of the eye by the intravitreous injection is summarized in Journal of ocular pharmacology and therapeutics, (2001) 17/4, 393-401 as a review. However, the intravitreous injection is a method of administration which requires skilled procedure and is accompanied by a considerable pain. Accordingly, burdens on patients are heavy, and it is very hard to administer the drug plural times.

Unlike these methods of administration, a subconjunctival injection, of which procedure is relatively easy, hardly causes disorders of ophthalmic tissues and burdens on patients are light, compared with the intravitreous injection. A delivery of a drug to the posterior segments of the eye after the subconjunctival injection was reported (see Invest. Ophthalmol. Visual Sci. 18 (3) 250-255, 1979), but its half-life was remarkably short, and it is difficult to maintain a drug concentration in the posterior segment tissues for a long period. Accordingly, frequent administration is required in order to maintain the drug concentration in the tissues, but the frequent administration increases the burdens on patients.

In order to avoid the frequent administration, it is necessary to maintain the drug concentration for a long time.

One method of maintaining the drug concentration for a long time is known in which a polymer is used in a vehicle for gelation to increase viscosity of a preparation. The polymer which increases viscosity of the preparation is exemplified by hydroxypropylmethylcellulose, hydroxyethylcellulose, carboxyvinyl polymers, carboxymethylcellulose, methylcellulose, polyvinylpyrrolidone, polyethylene glycol, which are widely-used. In addition to these polymers, substances which gel by a change of a certain factor of a living body are also known. There is a thermosensitive gel, which gels by a change of temperature, an ion-sensitive gel, which gels by an ion, a pH-sensitive gel, which gels by a change of pH and the like. The thermosensitive gel is exemplified by a mixture consisting of methylcellulose, citric acid and polyethylene glycol, which is a liquid at a lower temperature than a body temperature and gels when the temperature is raised to the body temperature (see Japanese Patent No. 2729859) and a polymer mixture consisting of polycaprolactone and polyethylene glycol, which is a liquid at a higher temperature than a body temperature and gels when the temperature is lowered to the body temperature (see Japanese Laid-open Patent Publication No. 176016/1996). Applicable gelling agents are not limited to these examples.

As examples of DDS using gels in an ophthalmic field, there is a report in which a preparation comprising timolol dissolved in a thermosensitive gel was instilled, and thereby a timolol concentration in an aqueous humor was maintained (see Japanese Patent No. 2729859), and there is a report in which a preparation comprising mitomycin C dissolved in a thermosensitive gel vehicle was administered subconjunctivally, and the drug delivery to sclera and conjunctiva was studied. (See British Journal of Ophthalmology 1997; 81: 72-75.)

However, since the conventional techniques are not sufficient to maintain a drug concentration in posterior segment tissues for a long time, it was desired to develop DDS which can release a drug gradually to the posterior segment so as to maintain an effective concentration of the drug for a long time.

### Disclosure of the Invention

The present inventors first focused attention on the fact that when a pharmaceutical composition comprising a drug and a vehicle is administered subconjunctivally, a depot is formed from the vehicle, and precisely studied a method of releasing the drug gradually from the depot, thereby enabling an effective concentration of the drug to be maintained.

As a result, it was found that excellent DDS is obtained by incorporating the drug into the vehicle which is in the form of gel at least after subconjunctival administration in a state where the drug is not dissolved in the vehicle instantaneously.

Namely, DDS according to the present invention is a drug delivery system to a posterior segment wherein a pharmaceutical composition comprising a drug and a vehicle is administered subconjunctivally to form a depot out of the vehicle, and thereby the drug is gradually released from the depot to enable an effective concentration of the drug to be maintained, the pharmaceutical composition comprising the vehicle which is in the form of gel subconjunctivally (namely, at least after subconjunctival administration) and the drug suspended in the vehicle.

A subconjunctival injection according to the present invention is an injection wherein the drug is suspended in the vehicle which is in the form of gel subconjunctivally (namely, at least after subconjunctival administration), the vehicle of the injection forms the depot subconjunctivally, and the drug is released gradually from the depot, thereby enabling the drug concentration in posterior segment tissues to be maintained.

The phrase "at least after subconjunctival administration" means that the vehicle can be but need not be in the form of gel before subconjunctival administration (including on administration), but the vehicle is necessarily in the form of gel after subconjunctival administration.

The depot in the present invention means that the pharmaceutical composition does not disperse in tissues over a long period, i.e., one week or longer, and is stored in tissues in a collective state, namely that the drug is stored in the vehicle which is in the form of gel in the tissues over the above-mentioned period, and that depot serves as a storage warehouse formed out of the vehicle which is in the form of gel in the tissues, and the drug is stored therein over the above-mentioned period.

The vehicle to be used in the present invention is a vehicle which is in the form of gel subconjunctivally. Because of such a form the vehicle stays in conjunctival tissues and serves as the warehouse (depot) of the drug.

The vehicle of the present invention can be in the form of gel on administration, and it can be in the form of solution on administration and after administration it can be in the form of gel subconjunctivally. The vehicle which is in the form of gel on administration contains a gel of polymer. Examples of polymers for gelation are hydroxypropylmethylcellulose, hydroxyethylcellulose, carboxyvinyl polymers, carboxymethylcellulose, methylcellulose, polyvinylpyrrolidone, polyethylene glycol and the like. The vehicle which is in the form of solution on administration and is in the form of gel subconjunctivally after administration contains a polymer which gels subconjunctivally. Examples of polymers which gel subconjunctivally are polymers which gel by a change of temperature, polymers which gel by an ion, polymers which gel by a change of pH and the like. The polymers which gel by the change of temperature are preferably polymers which gel at a conjunctiva temperature (about 37°C). The polymers which gel by the ion are preferably polymers which gel by an ion existing subconjunctivally such as a sodium ion or a calcium ion. The polymers which gel by the change of pH are preferably polymers which gel at conjunctiva pH (around neutrality). Using a polymer which satisfies these conditions, the polymer is in the form of solution on subconjunctival injection and gels after injection to form the depot.

Specific examples of polymers which gel by the change of temperature are gels described in Japanese Patent No. 2729859, registered trademark "Pluronic" (manufactured by Asahi Denka Co., Ltd.), registered trademark "ReGel" (manufactured by Macro Med Co., Ltd.) and the like. For example, the gel described in Japanese Patent No. 2729859 is a mixture of 1.4% by weight of methylcellulose, 3.5% by weight of citric acid and 2% by weight of polyethylene glycol and gels at 32°C or higher.

Specific examples of polymers which gel by the ion are gellan gum, sodium alginate and the like. Gellan gum gels in the presence of a cation such as a sodium ion. Sodium alginate gels in the presence of a divalent or higher cation such as a calcium ion. Gellan gum is usually used in combination with trometamol or mannitol.

Examples of polymers which gel by the change of pH are a mixture of polyacrylic acid and hydroxypropylmethylcellulose and the like. Though these polymers are in the form of low-viscosity liquids at pH of 4.0, they gel at pH of 7.4 (J pharm Sci 1995 Mar; 84 (3): 344-8).

The above-mentioned polymers can be used in combination of them.

According to the present invention, incorporating the drug into the vehicle which is in the form of gel at least after subconjunctival administration in the state where the drug is not dissolved in the vehicle instantaneously, an excellent sustained release effect and an excellent effective concentration-maintaining effect of the drug are obtained. When the drug is hardly water-soluble, the drug is incorporated into a vehicle to form a suspension in the vehicle. When the drug is relatively water soluble, it can be converted into a hardly water-soluble prodrug such as an ester. The drug can be prevented from dissolving instantaneously in the vehicle which is in the form of gel by incorporating the drug made in the form of microspheres into a vehicle, too.

According to DDS of the present invention, it is preferable to be administered subconjunctivally in the form of an injection.

The posterior segments of the eye in the present invention mean inner tissues of eyes such as a retina, choroid, an optic nerve, a vitreous body and a crystalline lens.

As described under the item of "Background Art", the drugs are hardly delivered to the posterior segments of the eye such as a retina, choroid and an optic nerve. Even if the drugs are delivered to the posterior segments of the eye, it is very difficult to maintain a drug concentration in those tissues.

In view of this, an intravenous injection, oral administration and an intravitreous injection are attempted to administer the drugs for the diseases of the posterior segments of the eye. However, the intravenous injection and the oral administration can deliver only a very small amount of drugs to the posterior segments of the eye which are target sites, and sometimes causes unexpected strong systemic actions (side effects) of the drugs.

In the case of the intravitreous injection, since the drug is directly injected into eyes, the amount of the drug to be delivered to the posterior segments of the eye is larger than those of the intravenous injection and the oral administration. However, the intravitreous injection is a method of administration which requires skilled procedure and is accompanied by a considerable pain. Accordingly, burdens on patients are heavy, and it is very difficult to administer the drug plural times.

Unlike these methods of administration, according to DDS of the present invention the pharmaceutical composition is administered by the subconjunctival injection. Therefore, the procedure is relatively easy, disorders of ocular tissues are hardly caused and burdens on patients are light compared with a intravitreous injection.

Examples of posterior segment diseases targeted by DDS of the present invention are inflammation due to various causes, viral or bacterial infections, diseases due to angiogenesis or vascular permeability augmentation of a retina-choroid and optic nerve disorders due to glaucoma. Further specific examples of diseases are uveitis, cytomegalovirus retinitis, age-related macular degeneration, diabetic retinopathy, proliferative vitreoretinopathy, retinal detachment, pigmentary retinal degeneration, contraction and visual field defect accompanying glaucoma and the like.

The drugs to be used in the present invention can be any drugs which are effective for treatment or prevention of the above-mentioned posterior segment diseases and are not particularly limited. Specific examples thereof are given below.

Examples of drugs are steroids such as betamethasone, dexamethasone, triamcinolone, prednisolone, fluorometholone, hydrocortisone and progesterone; anti-inflammatories such as bromofenac and diclofenac; cytokine inhibitors such as TNF-α inhibitors, PDE-IV inhibitors and ICE inhibitors; immunosuppressors such as ciclosporin and tacrolimus; antivirals such as ganciclovir, aciclovir and interferon·β; antimicrovials such as ofloxacin, clarithromycin and erythromycin; carcinostatic agents such as fluorouracil, methotrexate and MMP inhibitors; angiogenesis inhibitors such as endostatin, VEGF inhibitors, antisense oligonucleotide, PKC inhibitors, adhesion factor inhibitors and vascular resting steroid; neural protectants-neural nutrition factors such as MK-801, timolol, creatine, taurine and BDNF.

The present invention is characterized in that the drug is contained in the vehicle in a suspension state. Water-solubility and a concentration of the drug determine whether or not the drug can be suspended in the vehicle. When the drug is hardly water-soluble, the drug can be suspended in the vehicle excepting a case where the drug concentration in the vehicle is low. For example, as will be shown in Examples later, when 1% by weight of betamethasone is contained, it can be suspended in the vehicle.

When the water-solubility of the drug is high or when a concentration of the hardly water-soluble drug is low and the drug cannot be suspended in the vehicle as it is, the drug is converted into its prodrug to render it hardly water-soluble, or the drug is encapsulated in nanospheres or microspheres, so that it can be suspended in the vehicle.

Specific examples of methods of hardly water-soluble converting the drug into its prodrug to render it hardly water-soluble are a method of converting insulin into zinc insulin to render it hardly water-soluble, and a method of converting penicillin into hardly water-soluble procaine penicillin by chemically modifying a hydrophilic group of penicillin.

To encapsulate the drug in nanospheres or microspheres, special methods are not necessary, and widely-used methods can be used which are exemplified by a grinding method using a mill, a phase separation method (a coacervation method), a spray drying method, a supercritical fluid method, an interfacial deposition method, an interfacial reaction method and the like. More specific examples of methods are a submerged drying method, which is an interfacial deposition method (J. Control. Release, 2, 343-352, (1985)), an interfacial polymerization method, which is an interfacial reaction method (Int. J. Pharm., 28, 125-132 (1986)) and a self-emulsification solvent diffusion method (J. Control. Release, 25, 89-98 (1993)). An appropriate process for production can be selected among these processes for production considering the particle diameter of the fine particles, the kind, properties or a content of the contained drug or the like.

According to DDS of the present invention, it is preferable to be administered subconjunctivally in the form of the injection. The DDS can be prepared using a widely-used preparation technique of the injection. One example of the processes for preparing DDS is briefly described below.

First, a gel of polymer or a polymer which is expected to gel subconjunctivally is added to a solvent to give a vehicle. The solvent can be any physiologically acceptable solvent and is preferably distilled water for injection. A preferred concentration of the polymer is a concentration such that it has a viscosity at which it is not difficult to inject it before administration and which is sufficient to form the depot after administration. Specifically the concentration is 0.5 to 30% by weight though it varies depending on the kind of polymer.

The hardly water-soluble drug is added to the vehicle, and the drug is dispersed and suspended uniformly to prepare the injection. To suspend the drug in the vehicle, special methods are not necessary and widely-used methods can be used. For example, betamethasone is added to the vehicle, and ground sufficiently in a mortar to disperse it in the vehicle. Similarly, the drug which is encapsulated in microspheres are added to the vehicle, and dispersed and suspended uniformly in the vehicle to prepare the injection. A liquid drug is emulsified instead of being suspended. To emulsify the drug, widely-used methods can be used which are exemplified by a surface chemical emulsification method, a mechanical emulsification method, a membrane emulsification method and the like.

Additives to be usually used for the injection are used for the vehicle of the present invention. Additives such as an osmotic pressure adjusting agent such as sodium chloride and a buffer such as sodium phosphate can be used.

The drug delivery system of the present invention is used for treatment or prevention of diseases of posterior segments of the eye, namely a retina, a choroid, an optic nerve, a vitreous body and a crystalline lens. Specific examples of diseases are inflammation due to various causes, viral or bacterial infections, diseases due to angiogenesis or vascular permeability augmentation of a retina-choroid and optic nerve disorders due to glaucoma. Further specific examples of diseases are uveitis, cytomegalovirus retinitis, age-related macular degeneration, diabetic retinopathy, proliferative vitreoretinopathy, retinal detachment, pigmentary retinal degeneration, contraction and visual -field defect accompanying glaucoma and the like.

Effects of the present invention will be described later in detail under the item of "drug concentration in retina-choroid measurement tests". Administering the preparations containing betamethasone suspended in various gel vehicles subconjunctivally and measuring a drug concentration in a retina-choroid, it was confirmed that the drug concentration in the retina-choroid is maintained.

The preparations in the drug delivery system of the present invention are administered subconjunctivally. The subconjunctival administration can be carried out using an ordinary subconjunctival injection. The procedure of the subconjunctival injection is relatively easy, and the burdens on patients are light as described under the item of "Background Art".

Further, since the drug can be efficiently delivered to the posterior segments of the eye such as a retina, a choroid and an optic nerve by using the system of the present invention, a dosage of the drug can be reduced, and consequently side effects can be reduced.

### Best Mode for Carrying out the Invention

Examples of preparations to be used for DDS of the present invention and results of drug kinetic tests using DDS of the present invention are illustrated below.

### 1. Preparations

Specific examples of preparations which can be used for a drug delivery system of the present invention are illustrated below.

### Preparation Example 1 (Betamethasone-thermosensitive gel suspension)

Trisodium citrate dihydrate (1.75 g) and polyethylene glycol 400 (1.0 g) are dissolved in ultrapure water (50 ml) heated at about 70°C. After the dissolution, methylcellulose (0.7 g) is added to the solution little by little with stirring to disperse it uniformly. The obtained dispersion is stirred in a water bath cooled with ice until it becomes colorless and transparent. Then the temperature is returned to room temperature, and a small amount of 1 N hydrochloric acid is added thereto to adjust pH to 6.5. A thermosensitive gel suspension is prepared in this manner.

To the thermosensitive gel suspension (10 ml), betamethasone (0.1 g) is added, and ground sufficiently in a mortar to disperse it uniformly.

### Preparation Example 2 (Betamethasone-ion-sensitive gel suspension)

Trometamol (0.091 g) and D-(-)-mannitol (4.5 g) are dissolved in ultrapure water (about 80 ml) heated at about 70°C. To the obtained solution, gellan gum (0.6 g) is added little by little with stirring to dissolve it. Then ultrapure water is added thereto so that the total volume is 100 ml. An ion-sensitive gel suspension is prepared in this manner.

To the ion-sensitive gel suspension (10 ml), betamethasone (0.1 g) is added, ground sufficiently in a mortar, and then dispersed uniformly with a hybrid mixer.

### Preparation Example 3 (Betamethasone-methylcellulose gel suspension)

Ultrapure water (50 ml) was heated at about 70°C, and methylcellulose (0.7 g) was added thereto little by little with stirring to disperse it uniformly. The obtained dispersion is stirred in an ice-cold water bath until the it becomes colorless and transparent. A methylcellulose gel suspension is prepared in this manner.

To the methylcellulose gel suspension (10 ml), betamethasone (100 mg) is added, and ground sufficiently in a mortar to disperse it uniformly.

### Preparation Example 4 (Microsphere fluorouracil-thermo sensitive gel suspension)

Fluorouracil (0.5 g) and polylactic acid (4.5 g) having weight-average molecular weight of 20,000 are dissolved in acetic acid (200 ml). Acetic acid is removed by freeze-drying to give a uniform mixture of fluorouracil and polylactic acid. The mixture is molten at about 100°C, and the melt is shaped into needles. The obtained needles are ground with a mill to form fine particles. The formed fine particles are sieved to give fluorouracil-containing microspheres having a particle diameter of 10 to 75 µm.

The fluorouracil-containing microspheres (0.1 g) are added to the thermosensitive gel suspension (10 ml) prepared according to Preparation Example 1, and ground sufficiently in a mortar to disperse it uniformly.

### 2. Measurement of drug concentration in retina-choroid

Using the betamethasone-gel suspensions of Preparation Examples 1 to 3, a betamethasone concentration in a retina-choroid was measured according to the method below. As a control, using a betamethasone suspension, a betamethasone concentration in retina-chorold in the betamethasone-gel suspension administration group was compared with that of a betamethasone suspension administration group. The betamethasone suspension was prepared by suspending betamethasone in a solvent (a solution containing 0.4% by weight of polysorbate 80 and 2.6% by weight of glycerin) so that a betamethasone concentration was 1% by weight. The living body-sensitive polymer-containing betamethasone suspensions of Preparation Examples 1 to 3 were prepared so that the betamethasone concentration was 1% by weight, which is the same as that of the betamethasone suspension.
1) An oxybuprocaine hydrochloride ophthalmic solution (0.5% by weight) was instilled into both eyes of Japanese white rabbits to anesthetize the eye surfaces.
2) The polymer-containing betamethasone suspension (1% by weight) was subconjunctivally administered to an upper portion of conjunctiva in an amount of 50 µl per eye with a syringe equipped with a 27 G needle. A dosage of betamethasone was about 500 µg. For a control group, the betamethasone suspension (1% by weight) was subconjunctivally administered to an upper portion of conjunctiva in an amount of 50 µl per eye with the syringe equipped with the 27 G needle.
3) A part of the rabbits was killed on 2nd day and the rest was killed on 7th day after administration respectively. After enucleation of eyeball, the retina-choroids were recovered. Then betamethasone concentrations in the retina-choroids were measured by high performance liquid chromatography.

Results of changes in drug concentration with time are shown in Table 1. (The values in Table 1 are the average of four eyes.) As apparent from Table 1, in the case of the betamethasone suspension, the betamethasone concentration in the retina-choroid was about 0.72 µg/g tissue after two days, but the concentration after seven days was below the detection limit. To the contrary, in the case of the betamethasone-thermosensitive gel suspension, the betamethasone concentration in the retina-choroid was about 10.55 µg/g tissue even after seven days, in the case of the betamethasone-ion-sensitive gel suspension, the betamethasone concentration in the retina-choroid was about 1.02 µg/g tissue even after seven days, and in these two case, the effective drug concentrations in the retina-choroid were maintained. Even in the case of the betamethasone-methylcellulose gel suspension, the concentration was about 1.30 µg/g tissue after seven days, and the effective drug concentration in the retina-choroid was maintained.

**Table 1**

| Betamethasone concentration in retina-choroid (µg/g tissue) | | |
|---|---|---|
| | After two days | After seven days |
| Control group (Betamethasone suspension) | 0.72 | ≦ Detection limit |
| Betamethasone-thermosensitive gel suspension | 3.78 | 10.55 |
| Betamethasone-ion-sensitive gel suspension | 0.82 | 1.02 |
| Betamethasone-methylcellulose gel suspension | 6.14 | 1.30 |

The values in the table are the average of three to four eyes. The detection limit is about 0.05 µg/g tissue.

### Industrial Applicability

The present invention can provide excellent DDS to posterior segments of the eye by subconjunctival administration.

## Claims

1. A drug delivery system to a posterior segment
wherein a pharmaceutical composition comprising a drug and a vehicle is administered subconjunctivally to form a depot out of the vehicle, and thereby the drug is gradually released from the depot to enable an effective concentration of the drug to be maintained, the pharmaceutical composition comprising the vehicle which is in the form of gel subconjunctivally and the drug suspended in the vehicle.

2. The drug delivery system as claimed in claim 1,
wherein a dosage form of the pharmaceutical composition is an injection.

3. The drug delivery system as claimed in claim 1,
wherein the vehicle is in the form of gel on administration or a vehicle which is in the form of solution on administration and in the form of a gel subconjunctivally after administration.

4. The drug delivery system as claimed in claim 3,
wherein the vehicle is a thermosensitive gel, an ion-sensitive gel or a pH-sensitive gel.

5. The drug delivery system as claimed in any one of claims 1 to 4, wherein the drug is a hardly water-soluble drug.

6. The drug delivery system as claimed in claim 5;
wherein the hardly water-soluble drug is a drug which has lipid-solubility by which the drug is not soluble in the vehicle and can be suspended in the vehicle.

7. The drug delivery system as claimed in claim 5,
wherein the hardly water-soluble drug is a drug which is made hardly water-soluble by converting a water-soluble drug into its prodrug.

8. The drug delivery system as claimed in any one of claims 1 to 4, wherein the drug is a water-soluble drug, the drug is encapsulated in a nanosphere or a microsphere, then it is suspended in a vehicle which is in the form of gel subconjunctivally to give a pharmaceutical composition, and the composition is administered subconjunctivally.

9. A subconjunctival injection comprising a drug suspended in a vehicle which is in the form of gel subconjunctivally, wherein the vehicle of the injection forms a depot subconjunctivally, and the drug is released gradually from the depot, thereby enabling a drug concentration in a posterior segment tissue to be maintained.

10. The drug delivery system as claimed in claim 1 and the subconjunctival injection as claimed in claim 9,
wherein the posterior segment is a retina, a choroid, an optic nerve, a vitreous body or a crystalline lens.

11. The drug delivery system as claimed in claim 1 and the subconjunctival injection as claimed in claim 9,
wherein the drug is a drug for treatment or prevention of a disease of a retina, a choroid, an optic nerve, a vitreous body or a crystalline lens.

12. The drug delivery system as claimed in claim 1 and the subconjunctival injection as claimed in claim 9,
wherein the drug is an anti-inflammatory, an immunosuppressor, an antiviral, an anticancer drug, an angiogenesis inhibitor, an optic neural protectant, an antimicrovial or an antifungal agent.
